# EUROPEAN PATENT SPECIFICATION

(11) **EP 2 612 587 B1**
(45) Date of publication and mention of the grant of the patent: **03.09.2014**
(21) Application number: 12808143.7
(22) Date of filing: 13.06.2012
(51) Int. Cl.: A61B 1/00, A61B 5/00

(54) **OPTICAL MEASUREMENT APPARATUS**
OPTISCHE MESSVORRICHTUNG
DISPOSITIF DE MESURE OPTIQUE

(30) Priority: 07.07.2011 US 201161505396 P
(43) Date of publication of application: 10.07.2013
(73) Proprietor: Olympus Medical Systems Corp., Tokyo 151-0072 (JP)
(72) Inventor: KAMIMURA, Kenji, Tokyo 151-0072 (JP); GONO, Kazuhiro, Tokyo 151-0072 (JP)
(74) Representative: Schicker, Silvia
(86) International application number: PCT/JP2012/065164
(87) International publication number: WO 2013/005547

(56) References cited:
- JP-A- 2006 095 166
- JP-A- 2006 341 078
- JP-A- 2009 537 014
- JP-A- 2010 063 839
- JP-A- 2010 200 883
- COTHREN R M ET AL: "Gastrointestinal tissue diagnosis by laser-induced fluorescence spectroscopy at endoscopy", GASTROINTESTINAL ENDOSCOPY, ELSEVIER, NL, vol. 36, no. 2, 1 March 1990 (1990-03-01), pages 105-111, XP025856502, ISSN: 0016-5107, DOI: 10.1016/S0016-5107(90)70961-3 [retrieved on 1990-03-01]

## Description

### Field

The present invention relates to an optical measurement apparatus for performing spectrometry of returned light reflected or scattered by body tissue to obtain a characteristic value of the body tissue. Background

In recent years, there is known a measurement method of measuring an optical property of body tissue while a probe leading end makes direct contact with the body tissue by inserting a probe into a forceps channel of an endoscope for observing internal organs such as digestive organs and projecting the probe leading end from the endoscope.

For example, there has been proposed an optical measurement apparatus in which properties of body tissue such as blood circulation in the body tissue, a hemodynamic status, and a hemoglobin amount variation are measured by irradiating near infrared light onto the body tissue and measuring the near infrared light passing through the body tissue or the near infrared light reflected at an internal side of the body tissue (for example, refer to Patent Literature 1).

In addition, there has been proposed an optical measurement apparatus using a low-coherence enhanced backscattering (LEBS) technique for detecting properties of body tissue by irradiating low-coherence white light having a short spatial coherence length from the probe leading end onto the body tissue and measuring a distribution of the scattering light intensity from a plurality of angles using a plurality of light receiving fibers (For example, refer to Patent Literature 2 and Patent Literature 3)

### Citation List

### Patent Literature

Patent Literature 1: Japanese Patent Application Laid-open No. 2010-104586
Patent Literature 2: International Patent Publication No. WO2007/133684
Patent Literature 3: U.S. Patent Application Laid-open No. 2008/0037024

### Summary

### Technical Problem

In an optical measurement apparatus, if a large noise caused by endoscopic illumination light is imposed on a measurement value, optical properties of body tissue cannot be obtained precisely, and as a result, detection accuracy of properties of body tissue is reduced. In the optical measurement apparatus disclosed in Patent Literature 2, for example, since the white light that is the same as the illumination light of the endoscope is used for measurement, the endoscopic illumination light may cause the noise. Therefore, if too much illumination of the white light from the endoscope is included in the measurement value, an effective measured value may not be obtained.

The present invention has been made in view of the forgoing, and an object of the present invention is to provide an optical measurement apparatus that can obtain a measurement value which is low in noise caused by the endoscopic illumination light.

### Solution to Problem

To solve the above problems and achieve the object, an optical measurement apparatus of the invention performs spectrometry of returned light reflected or scattered by body tissue to obtain a characteristic value of the body tissue. The optical measurement apparatus includes: a probe having an irradiation fiber that propagates light supplied from a base end and irradiates the light from a leading end and a plurality of light receiving fibers that propagate light incident from leading ends and output the light from base ends; a light source unit that generates white light to be irradiated onto the body tissue and supplies the white light to the irradiation fiber; a measurement unit that performs spectrometry for the returned light from the body tissue output from each of the light receiving fibers at a predetermined measurement timing; and a control unit that determines whether or not a measurement value that is measured by the measurement unit is equal to or smaller than a predetermined threshold value, and causes the light source unit to perform a light emission process for obtaining a characteristic value of the body tissue for a predetermined time and causes the measurement unit to perform a spectrometry process for obtaining a characteristic value of the body tissue for the predetermined time if the determining unit determines that the measurement value measured by the measurement unit is equal to or smaller than the predetermined threshold value.

The optical measurement apparatus according to the invention further includes a storage unit that stores data for obtaining a characteristic value of the body tissue. The control unit causes the storage unit to store the spectrometric result measured by the measurement unit as data for a characteristic value of the body tissue if it is determined that the measurement value, initially measured by the measurement unit after the light source unit completes the light emission process for obtaining a characteristic value, is equal to or smaller than the predetermined threshold value.

The optical measurement apparatus according to the invention further includes an input unit that inputs instruction information for instructing to obtain data for obtaining a characteristic value of the body tissue. The control unit determines whether or not the measurement value measured by the measurement unit is equal to or smaller than a predetermined threshold value when the instruction information is input by the input unit.

In the optical measurement apparatus according to the invention, the probe is inserted into an insertion portion of an endoscope inserted into an inner side of a subject, and a leading end is projected from the endoscope.

The optical measurement apparatus according to the invention further includes: an imaging unit that captures an image at the leading end of the probe projected from the endoscope; and a projection length computation unit that computes a projection length of the probe from the endoscope using a photographic image of the leading end of the probe captured by the imaging unit. The control unit causes the light source unit to perform the light emission process and causes the measurement unit to perform the spectrometry, if the measurement value measured by the measurement unit is equal to or smaller than a predetermined threshold value, and the projection length of the probe computed by the projection length computation unit is within a predetermined allowable range.

In the optical measurement apparatus according to the invention, a plurality of patterns having predetermined regularity are formed in the leading end of the probe, and the projection length computation unit computes the projection length of the probe from the endoscope by measuring the pattern viewed on the photographic image.

In the optical measurement apparatus according to the invention, the storage unit stores a diameter of the probe, and the projection length computation unit computes the projection length of the probe from the endoscope based on the diameter of the probe stored in the storage unit and a ratio between a diameter of the probe viewed on the photographic image and a length of the probe viewed on the photographic image.

### Advantageous Effects of Invention

According to the optical measurement apparatus of the invention, if the measurement value is equal to or smaller than a predetermined threshold value, that is, only when noise included in the measurement result is insignificant, the light emission process for obtaining a characteristic value of the body tissue and spectrometry for obtaining characteristic value of the body tissue are performed. Therefore, it is possible to reliably obtain a measurement value having little noise caused by the endoscopic illumination light.

### Brief Description of Drawings

FIG. 1 is a schematic diagram illustrating a schematic configuration of an optical measurement apparatus according to a first embodiment.
FIG. 2 is a diagram illustrating a configuration of an endoscope system and how a probe is installed in the optical measurement apparatus.
FIG. 3A is a diagram illustrating a measurement state of the optical measurement apparatus of FIG. 1.
FIG. 3B is a diagram illustrating a measurement state of the optical measurement apparatus of FIG. 1.
FIG. 4 is a diagram illustrating time dependence of the measurement result of the measurement unit and a light amount emitted from the light source unit of FIG. 1.
FIG. 5 is a flowchart illustrating an optical measurement processing sequence of the optical measurement apparatus of FIG. 1.
FIG. 6 is a schematic diagram illustrating a schematic configuration of an optical measurement apparatus according to a second embodiment.
FIG. 7 is a flowchart illustrating an optical measurement processing sequence of the optical measurement apparatus of FIG. 6.
FIG. 8 is a schematic diagram illustrating a schematic configuration of an optical measurement apparatus according to a third embodiment.
FIG. 9 is a perspective view illustrating a probe leading end of FIG. 8.
FIG. 10 is an exemplary photographic image of a processing target of an image processing unit of FIG. 8.
FIG. 11 is a flowchart illustrating an optical measurement processing sequence of the optical measurement apparatus of FIG. 8.
FIG. 12 is a perspective view illustrating another example of the probe leading end of FIG. 8.
FIG. 13 is an exemplary photographic image of a processing target of the image processing unit of FIG. 8.
FIG. 14 is a schematic diagram illustrating a schematic configuration of an optical measurement apparatus according to a fourth embodiment.
FIG. 15 is a flowchart illustrating an optical measurement processing sequence of the optical measurement apparatus of FIG. 14.

### Description of Embodiments

Hereinafter, an exemplary optical measurement apparatus using LEBS technique will be described in detail as preferable embodiments of an optical measurement apparatus according to the present invention with reference to the accompanied drawings. The invention is not limited to the embodiments described below. In the description of drawings, like reference numerals denote like elements. It is noted that the drawings are schematically provided, and thicknesses and widths of each element and ratios of each element may be different from those of the reality. Among the drawings, a portion having a different relationship or ratio from that of other drawings may be included.

### (First Embodiment)

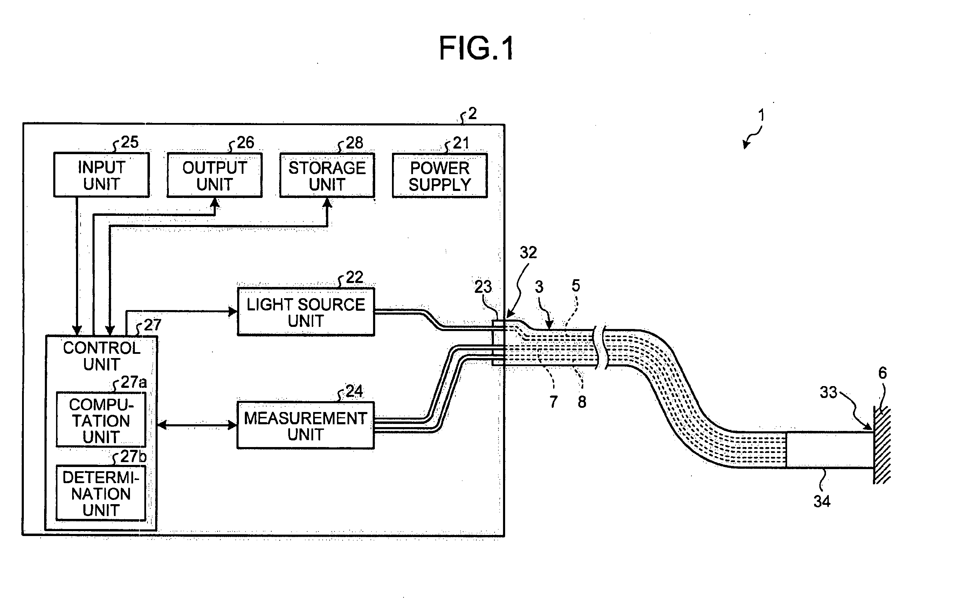

FIG. 1 is a schematic diagram illustrating a schematic configuration of an optical measurement apparatus according to a first embodiment of the invention. As illustrated in FIG. 1, an optical measurement apparatus 1 according to the first embodiment includes a main unit 2 that performs optical measurement for body tissue 6 as a measurement target and detects a property of the body tissue 6 and a measurement probe 3 inserted into a subject. The probe 3 has flexibility, and a base end 32 is detachably connected to the main unit 2 so that the light supplied from the base end 32 is emitted from a leading end 33 to the body tissue 6 using the connected main unit 2, and reflection light and scattering light incident from the leading end 33 as the returned light from the body tissue 6 are output from the base end 32 to the main unit 2.

The main unit 2 includes a power supply 21, a light source unit 22, a connector 23, a measurement unit 24, an input unit 25, an output unit 26, a control unit 27, and a storage unit 28.

The power supply 21 supplies electric power to each element of the main unit 2.

The light source unit 22 generates and outputs light to be irradiated onto the body tissue 6. The light source unit 22 includes white light-emitting diode (LED) that emits white light, a low-coherence light source such as a xenon lamp or a halogen lamp, and one or more lenses (not illustrated). The light source unit 22 supplies the low-coherence light irradiated onto an object to an irradiation fiber 5 of the probe 3 described below.

The connector 23 detachably connects the base end 32 of the probe 3 to the main unit 2. The connector 23 supplies the light emitted from the light source unit 22 to the probe 3 and outputs the returned light output from the probe 3 to the measurement unit 24.

The measurement unit 24 performs spectrometry for the returned light from the body tissue 6 as the light output from light receiving fibers 7 and 8 of the probe 3. The measurement unit 24 includes a plurality of spectrometers. The measurement unit 24 measures a spectral component, strength, and the like of the returned light output from the probe 3 and performs measurement on a wavelength basis. The measurement unit 24 outputs the measurement result to the control unit 27.

The input unit 25 is realized by a push-type switch and the like. The input unit 25 receives instruction information for instructing activation of the main unit 2 or various other types of instruction information by manipulating the switch and the like and inputs it to the control unit 27.

The output unit 26 outputs information regarding various processes in the optical measurement apparatus 1. The output unit 26 is realized by a display, a speaker, a motor, and the like so that information regarding various processes in the optical measurement apparatus 1 is output by outputting image information, audio information, or vibration.

The control unit 27 controls processing operations of each element of the main unit 2. The control unit 27 is realized by a CPU and semiconductor memory such as RAM. The control unit 27 controls operations of the main unit 2 by transmitting instruction information or data to each element of the main unit 2 and the like. The control unit 27 stores each measurement result from the measurement unit 24 having a plurality of measurement devices in the storage unit 28 described below. The control unit 27 includes a computation unit 27a and a determination unit 27b.

The computation unit 27a performs various types of computation processes based on the measurement result of the measurement unit 24 to compute the characteristic value associated with the property of the body tissue 6. The type of the characteristic value computed by the computation unit 27a and serving as a target to obtain is set depending on instruction information input from the input unit 25 through manipulation of an operator.

The determination unit 27b determines whether or not the received light amount measured by the measurement unit 24 is equal to or smaller than a predetermined threshold value. If the received light amount measured by the measurement unit 24 is equal to or smaller than a predetermined threshold value, the determination unit 27b causes the light source unit 22 to perform a light emission process for obtaining a characteristic value of body tissue 6 for a predetermined time and causes the measurement unit 24 to perform spectrometry for obtaining the characteristic value of the body tissue 6. If it is determined that the measurement value initially measured by the measurement unit 24 after the light source unit 22 completes the light emission process is equal to or smaller than the predetermined threshold value, the determination unit 27b causes the storage unit 28 to store the spectrometric result measured by the measurement unit 24 as data for the characteristic value of the body tissue 6 for the predetermined time.

The storage unit 28 stores optical measurement program for executing the optical measurement process in the main unit 2 and various types of information regarding the optical measurement process. The storage unit 28 stores various measurement results from the measurement unit 24. In addition, the storage unit 28 stores the characteristic value computed by the computation unit 27a.

The probe 3 has the base end 32 detachably connected to a predetermined connection unit of the main unit 2 and the leading end 33 making direct contact with the body tissue 6. The leading end 33 emits light supplied from the light source unit 22 and receives scattering light from a measurement target. If an LEBS technique is used, the probe 3 is provided with a plurality of light receiving fibers for receiving at least two scattering light beams having different scattering angles. Specifically, the probe 3 has a irradiation fiber 5 that propagates light from the light source unit 22 supplied from the base end 32 and irradiates the light from the leading end 33 onto the body tissue 6 and two light receiving fibers 7 and 8 that propagate scattering light and reflection light from the body tissue 6 incident from the leading end 33 and output the light to the base end 32. The leading ends of the irradiation fiber 5 and the light receiving fibers 7 and 8 are provided with a rod 34 having transparency. The rod 34 has a cylindrical shape such that distances between the surface of the body tissue 6 and the leading ends of the irradiation fiber 5 and the light receiving fibers 7 and 8 become constant. Although the probe 3 has two light receiving fibers 7 and 8 in the example of FIG. 1, the probe 3 may have three or more light receiving fibers if at least two or more scattering light beams having different scattering angles are received.

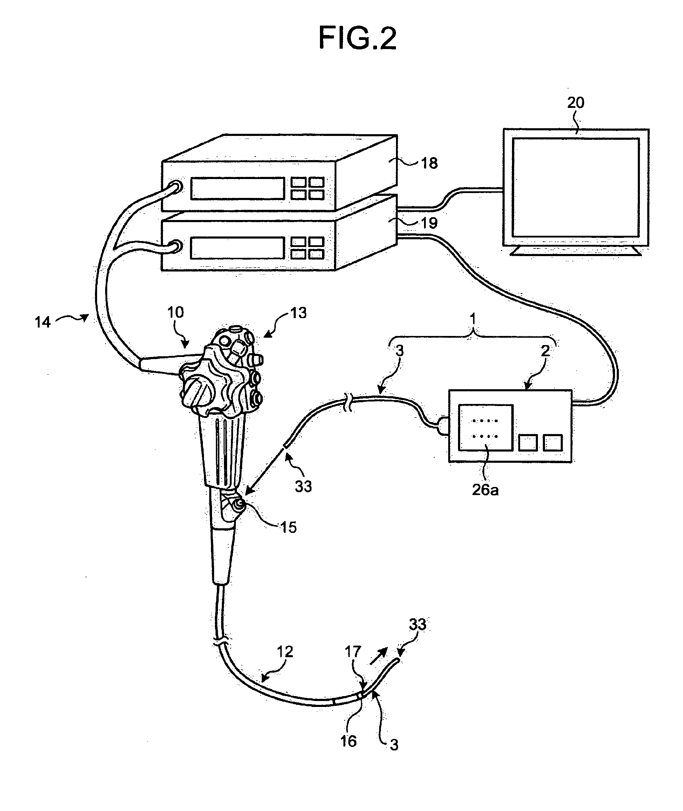

The optical measurement apparatus 1 is usually combined with an endoscope system for observing internal organs such as digestive organs. FIG. 2 illustrates a configuration of the endoscope system and how to install the probe 3 in the optical measurement apparatus 1. In FIG. 2, a flexible universal cord 14 extending from the lateral side of a manipulation unit 13 is connected to a light source device 18 and a signal processor 19 that processes the object image captured at a leading end portion 16 of an endoscope 10. The signal processor 19 is connected to a display 20. The display 20 displays various types of information regarding inspection, including an object image processed by the signal processor 19.

The probe 3 is inserted from a probe channel insertion hole 15 in the vicinity of the manipulation unit 13 of an out-body portion of the endoscope 10 inserted into a subject as indicated by the arrow. In addition, the leading end 33 of the probe 3 is projected from an aperture 17 of the leading end portion 16 passing through the internal side of an insertion portion 12 and connected to the probe channel as indicated by the arrow. As a result, the probe 3 is inserted into the internal side of the subject, and optical measurement is initiated.

A display screen 26a for outputting a determination result of the determination unit 27b, a characteristic value computed by the computation unit 27a, and the like, a switch serving as a part of the input unit 25, and the like are provided on a predetermined surface of the main unit 2. As illustrated in FIG. 2, the main unit 2 of the optical measurement apparatus 1 is connected to the signal processor 19, and various types of information processed by the optical measurement apparatus 1 may be output to the signal processor 19 and displayed on the display 20.

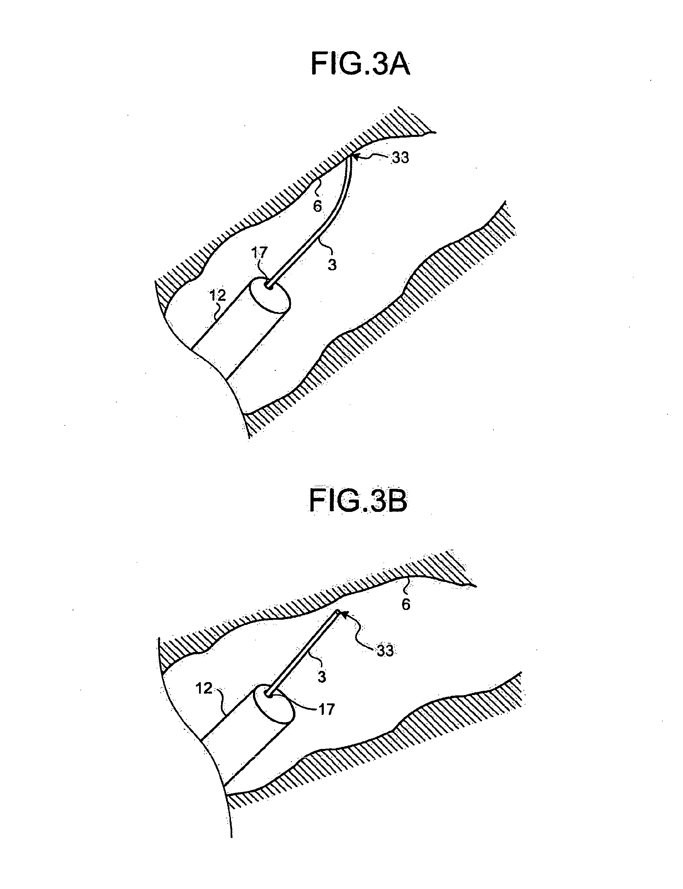

Here, in the optical measurement apparatus 1, if the leading end 33 of the probe 3 projected from the aperture 17 of the leading end of the insertion portion 12 of the endoscope 10 appropriately makes contact with the surface of the body tissue 6 in the hollow viscus as illustrated in FIG. 3A, it is possible to obtain a valid measurement value having little white illumination light from the endoscope 10 incident to the leading end of the probe 3 with little noise caused by the endoscope illumination. However, in general, it is difficult to fix the leading end 33 of the probe 3 to the measurement position on the body tissue 6 due to movement caused by pulsation or peristalsis during measurement of internal organs such as digestive organs. As illustrated in FIG. 3B, when it is difficult to stably fix the leading end 33 of the probe 3 on the surface of the body tissue 6 due to peristalsis of the internal organs and the like, the white illumination light from the endoscope 10 is easily incident from the leading end of the probe 3, and a measurement value has significant noise caused by the endoscope illumination. Therefore, the optical measurement apparatus may not reliably obtain a valid measurement value with little noise.

For this reason, in the optical measurement apparatus 1 according to the first embodiment, the light emission process and spectrometry for obtaining a characteristic value of the body tissue 6 are performed only when the measurement value measured in a state that only the endoscopic illumination light is irradiated is low as it can guarantee validity of the measurement value. As a result, it is possible to obtain a measurement value having little noise caused by the endoscopic illumination light.

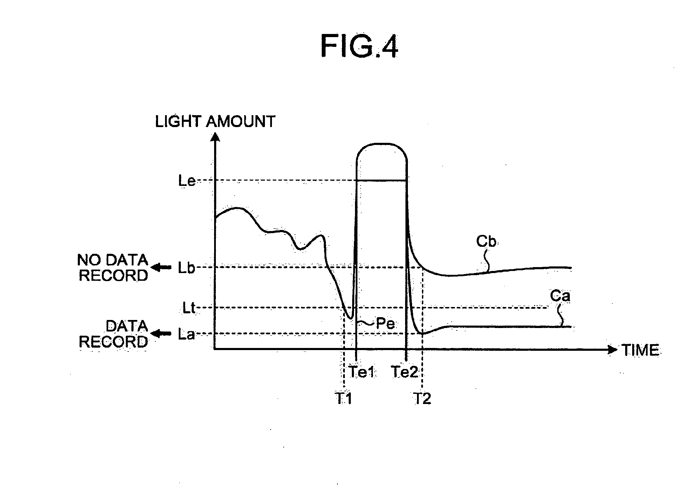

Specifically, according to the first embodiment, as illustrated in FIG. 4, the threshold value Lt is set depending on the light amount of the endoscopic illumination light that can be determined as it can guarantee validity of the measurement value for the actual body tissue 6. The determination unit 27b causes the measurement unit 24 to measure the amount of light output from at least any one of the light receiving fibers 7 and 8 at a predetermined timing in a state that only the endoscopic illumination light is irradiated. In this case, the measurement unit 24 may measure the light amounts for overall wavelengths set for the measurement process for obtaining a characteristic value of the body tissue 6 or may measure a light amount for only a predetermined wavelength.

Subsequently, if the measurement value from the measurement unit 24 at the time T1 is equal to or smaller than the threshold value Lt, the determination unit 27b causes the light source unit 22 to perform the light emission process for obtaining a characteristic value of the body tissue 6 and causes the measurement unit 24 to perform a measurement process for obtaining a characteristic value of the body tissue 6. The light source unit 22 generates and outputs pulse light having a certain strength Le as indicated in a curve Pe for a predetermined time from Te1 to Te2 as the light emission process for obtaining a characteristic value. The output time of the pulse light using the light source unit 22 may be set to a range between 1 millisecond and 1 second, and preferably, between 1 to 500 milliseconds.

Therefore, in the optical measurement apparatus 1, spectrometry and the light emission process for obtaining a characteristic value of the body tissue 6 are performed only when the measurement value of the received light amount measured in a state that only the endoscopic illumination light is irradiated is low as it can guarantee validity of the measurement value.

Then, when the light amount of the endoscopic illumination light incident to the light receiving fibers 7 and 8 is maintained to a level that validity of the measurement value for the actual body tissue 6 can be guaranteed, the measurement value of the received light amount using the measurement unit 24 after output generation of pulse light using the light source unit 22 is terminated is returned to a value equal to or smaller than the threshold value Lt similar to a case before the pulse light is output as indicated by the curve Ca. In comparison, when the light amount of the endoscopic illumination light incident to the light receiving fibers 7 and 8 is large sufficiently to fail to guarantee validity of the measurement value for the actual body tissue 6 so that the endoscopic illumination light is overlapped with the measurement value as significant noise, as indicated by the curve Cb of FIG. 4, the measurement value of the received light amount using the measurement unit 24 is greater than the threshold value Lt even after output generation of pulse light using the light source unit 22 is terminated.

Thus, if the measurement value La at the time T2 after output of pulse light using the light source unit 22 is generated is equal to or smaller than the threshold value Lt as indicated by the curve Ca, the determination unit 27b determines that the light amount of the endoscopic illumination light incident to the light receiving fibers 7 and 8 is set to a level capable of guaranteeing validity of the measurement value for the actual body tissue 6 so that the spectrometric result measured by the measurement unit 24 for the time Te1 to Te2 is stored in the storage unit 28 as data for a characteristic value of the body tissue 6. In comparison, if the measurement value Lb at the time T2 is greater than the threshold value Lt as indicated by the curve Cb, the determination unit 27b determines that the light amount of the endoscopic illumination light incident to the light receiving fibers 7 and 8 is large sufficient to fail to guarantee validity of the measurement value for the actual body tissue 6, so that the spectrometric result measured by the measurement unit 24 for the time Te1 to Te2 is not employed as data for a characteristic value of the body tissue 6 and is not stored in the storage unit 28.

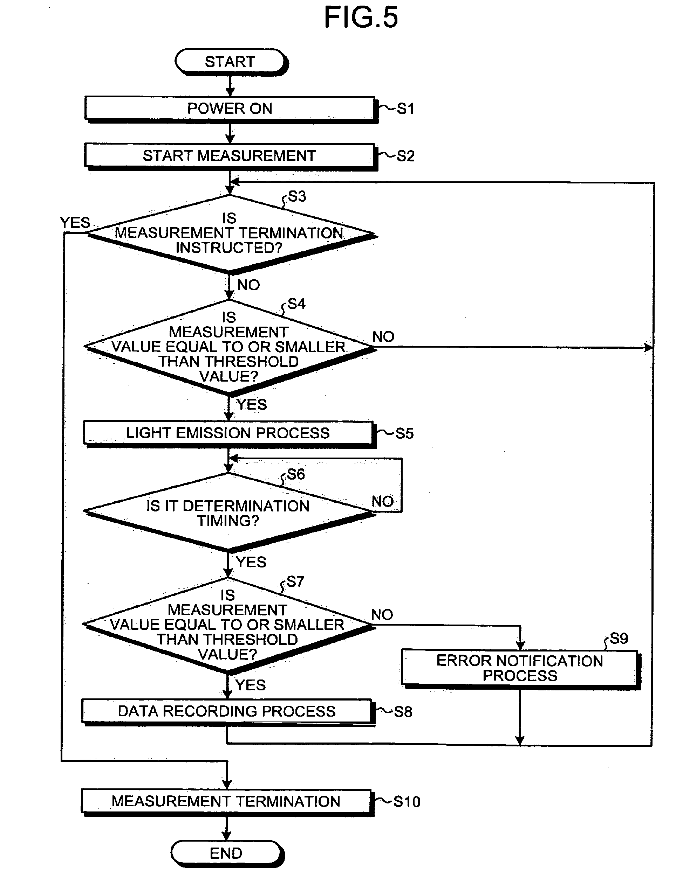

Next, a processing sequence of the optical measurement process of the optical measurement apparatus 1 will be described with reference to FIG. 5. FIG. 5 is a flowchart illustrating the optical measurement processing sequence in the optical measurement apparatus 1 of FIG. 1.

As illustrated in FIG. 5, the power supply of the optical measurement apparatus 1 is turned on (step S1), and the measurement unit 24 initiates measurement for the light output from at least any one of the light receiving fibers 7 and 8 (step S2). The measurement unit 24 performs a measurement process for every predetermined measurement timing and sequentially outputs the measurement value to the control unit 27. The measurement unit 24 performs the measurement process in the unit of time sufficiently shorter than the output time of pulse light from the light source unit 22 and sequentially outputs the measurement value to the control unit 27.

Subsequently, the determination unit 27b determines whether or not the measurement termination is instructed based on instruction information for instructing measurement termination from the input unit 25 (step S3). If it is determined that the measurement termination is instructed (YES in step S3), the determination unit 27b terminates the measurement process in the measurement unit 24 (step S10) to terminate the measurement process for the body tissue 6.

Otherwise, if it is determined that the measurement termination is not instructed (NO in step S3), the determination unit 27b determines whether or not the measurement value output from the measurement unit 24 is equal to or smaller than a predetermined threshold value (step S4). If the determination unit 27b determines that the measurement value output from the measurement unit 24 is not equal to or smaller than the predetermined threshold value (NO in step S4), the process returns to step S3.

Otherwise, if the determination unit 27b determines that the measurement value output from the measurement unit 24 is equal to or smaller than the predetermined threshold value (YES in step S4), the light source unit 22 performs a light emission process for obtaining a characteristic value of the body tissue 6 (step S5).

Then, the determination unit 27b determines whether or not it is the determination timing for determining whether or not the record of the measurement result measured during the light emission process in step S5 is appropriate (step S6). This determination timing is performed when a predetermined time elapses after the light emission process is terminated, and preferably, after an initial measurement process in the measurement unit 24 is terminated after the light emission process is terminated. If the determination unit 27b determines that it is not the determination timing (NO in step S6), the determination process in step S6 is repeated.

Otherwise, if the determination unit 27b determines that it is the determination timing (YES in step S6), it is determined whether or not the measurement value output from the measurement unit 24 during the determination timing is equal to or smaller than a predetermined threshold value (step S7).

If the determination unit 27b determines that the measurement value output from the measurement unit 24 during the determination timing is equal to or smaller than the predetermined threshold value (YES in step S7), it can be determined that the light amount of the endoscopic illumination light incident to the light receiving fibers 7 and 8 during the light emission process is maintained at a level capable of guaranteeing validity of the measurement value for the actual body tissue 6. For this reason, in this case, the determination unit 27b performs a data recording process for storing the spectrometric result measured by the measurement unit 24 during the light emission process in the storage unit 28 as data for a characteristic value of the body tissue 6 (step S8).

Otherwise, if the determination unit 27b determines that the measurement value output from the measurement unit 24 during the determination timing is not equal to or smaller than the predetermined threshold value (NO in step S7), that is, if it is determined that the measurement value exceeds the predetermined threshold value, it may be determined that the light amount of the endoscopic illumination light incident to the light receiving fibers 7 and 8 during the light emission process is overlapped with the measurement value so as to serve as significant noise as much as it fails to guarantee validity of the measurement value for the actual body tissue 6. For this reason, in this case, the determination unit 27b performs an error notification process for notifying the output unit 26 of an error message that the obtained measurement value is not valid (step S9). As the error notification process, the determination unit 27b may cause the output unit 26 to output a sound notifying a fact that the obtained measurement value is not valid, or a display screen notifying a fact that the obtained measurement value is not valid, or output both of the sound and the display screen. In addition, after step S8 or S9 is terminated, the process returns to step S3 so that the determination unit 27b determines whether or not the measurement termination is instructed.

In this manner, in the optical measurement apparatus 1 according to the first embodiment, if the measurement value is equal to or smaller than a predetermined threshold value, that is, only when the noise caused by the endoscopic illumination light included in the measurement result is insignificant, the light emission process for obtaining a characteristic value of the body tissue 6 and spectrometry for obtaining characteristic value of the body tissue 6 are performed. Therefore, it is possible to reliably obtain a measurement value having little noise.

In addition, in the optical measurement apparatus 1 according to the first embodiment, the spectrometric result measured during the light emission process is stored as data for the characteristic value of the body tissue 6 only when it is determined that the measurement value initially measured by the measurement unit 24 is equal to or smaller than a predetermined threshold value after the light emission process for obtaining a characteristic value is terminated. Therefore, it is possible to automatically obtain only the measurement value having the endoscopic illumination light influence sufficiently lowered to a level capable of guaranteeing validity.

### (Second Embodiment)

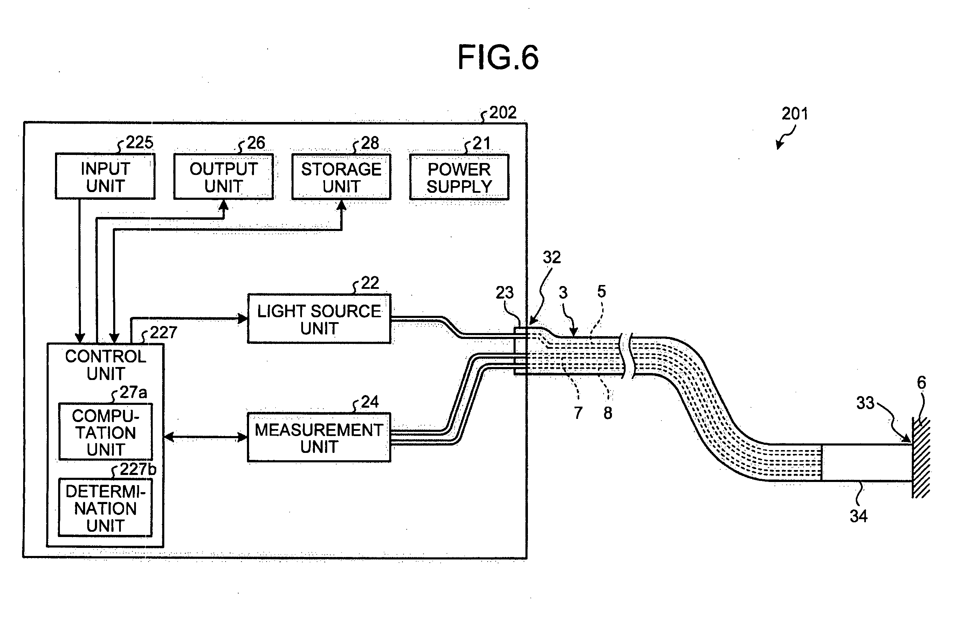

Next, a second embodiment will be described. FIG. 6 is a schematic diagram illustrating a schematic configuration of the optical measurement apparatus according to the second embodiment of the present invention.

As illustrated in FIG. 6, an optical measurement apparatus 201 according to the second embodiment has a main unit 202 instead of the main unit 2 of FIG. 1. The main unit 202 has an input unit 225 having the same function as that of the input unit 25 and receiving instruction information for instructing to obtain data for obtaining a characteristic value of the body tissue 6 instead of the input unit 25. In addition, the main unit 202 has a control unit 227 having the same function as that of the control unit 27 instead of the control unit 27. The control unit 227 has a determination unit 227b having the same function as that of the determination unit 27b and determining whether or not the measurement value measured by the measurement unit 24 is equal to or smaller than a predetermined threshold value when instruction information for instructing to obtain data for obtaining a characteristic value of the body tissue 6 is input from the input unit 225 instead of determination unit 27b.

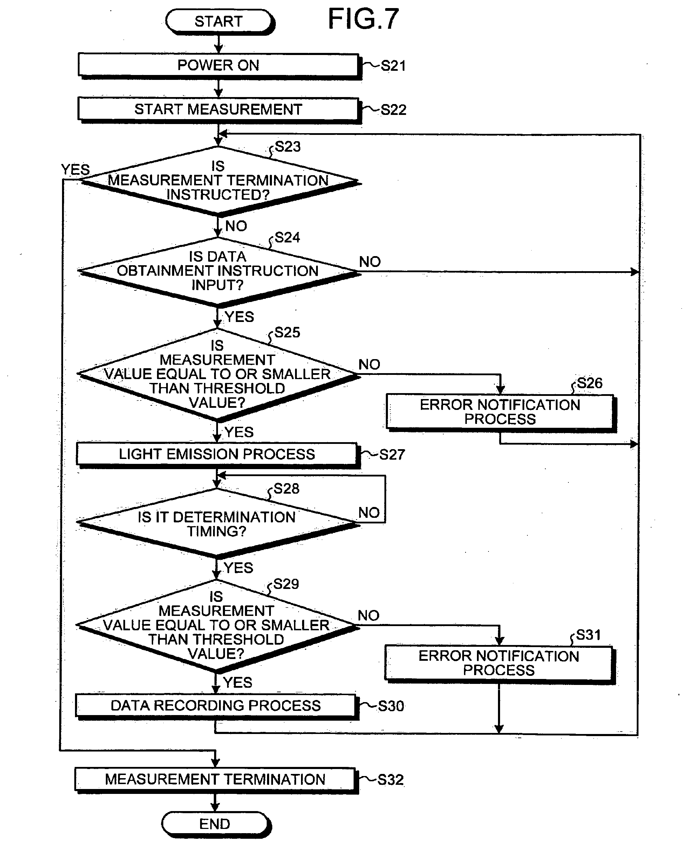

Next, a processing sequence of the optical measurement process of the optical measurement apparatus 201 will be described with reference to FIG. 7. FIG. 7 is a flowchart illustrating an optical measurement processing sequence of the optical measurement apparatus 201 of FIG. 6.

Steps S21 and S22 of FIG. 7 are similar to steps S1 and S2, respectively, of FIG. 5. Subsequently, similar to step S3 of FIG. 5, the determination unit 227b determines whether or not the measurement termination is instructed (step S23). If it is determined that the measurement termination is instructed (Yes in step S23), the measurement process in the measurement unit 24 is terminated (step S32). Otherwise, if it is determined that the measurement termination is not instructed (No in step S23), the determination unit 227b determines whether or not a data obtainment instruction for obtaining a characteristic value is input based on whether or not there is instruction information for instructing to obtain data for obtaining a characteristic value of the body tissue 6 from the input unit 225 (step S24). If the determination unit 227b determines that the data obtainment instruction for obtaining a characteristic value is not input (No in step S24), the process returns to step S23.

If it is determined that the data obtainment instruction for obtaining a characteristic value is input (Yes in step S24), similar to step S4 of FIG. 5, the determination unit 227b determines whether or not the measurement value output from the measurement unit 24 is equal to or smaller than a predetermined threshold value (step S25). If the determination unit 227b determines that the measurement value output from the measurement unit 24 is not equal to or smaller than the predetermined threshold value (No in step S25), an error notification process for causing the output unit 26 to notify a fact that the measurement may not be initiated (step S26) is performed, and then, the process returns to step S23.

If determination unit 227b determines that the measurement value output from the measurement unit 24 is equal to or smaller than the predetermined threshold value (Yes in step S25), similar to step S5 of FIG. 5, the light source unit 22 performs the light emission process for obtaining a characteristic value of the body tissue 6 (step S27).

Then, similar to step S6 of FIG. 5, the determination unit 227b determines whether or not it is a determination timing for determining whether or not the record of the measurement result measured during the light emission process is appropriate (step S28). If the determination unit 227b determines that it is not the determination timing (No in step S28), the determination process of step S28 is repeated. If the determination unit 227b determines that it is the determination timing (Yes in step S28), similar to step S7 of FIG. 5, the determination unit 227b determines whether or not the measurement value output from the measurement unit 24 during the determination timing is equal to or smaller than a predetermined threshold value (step S29).

If the determination unit 227b determines that the measurement value output from the measurement unit 24 during the determination timing is equal to or smaller than the predetermined threshold value (Yes in step S29), similar to step S8 of FIG. 5, the data recording process is performed for the spectrometric result measured by the measurement unit 24 during the light emission process (step S30). Otherwise, if the determination unit 227b determines that the measurement value output from the measurement unit 24 during the determination timing is not equal to or smaller than the predetermined threshold value (No in step S29), similar to step S9 of FIG. 5, an error notification process for causing the output unit 26 to notify a fact that the obtained measurement value is not valid is performed (step S31). In addition, after step S30 or S31 is terminated, the process returns to step S23, and the determination unit 227b determines whether or not the measurement termination is instructed.

In this manner, according to the second embodiment, the measurement value having significant noise and being overlapped is not obtained or recorded even when data obtainment for obtaining a characteristic value is instructed through manipulation of the input unit 225 from an operator. Therefore, it is possible to reliably obtain only the measurement value having little noise.

### (Third Embodiment)

Next, a third embodiment will be described. If a projection length of the probe from the leading end of the insertion portion of the endoscope is small, the endoscope illumination is still close even when the probe leading end appropriately makes contact with body tissue. Therefore, the light amount of the endoscopic illumination light incident to the probe leading end increases so that the endoscope illumination is overlapped with the measurement value as noise. Meanwhile, if the projection length of the probe from the leading end of the insertion portion of the endoscope is too large, the endoscope illumination becomes distant so that execution of the measurement process and the light emission process for obtaining a characteristic value is determined in a dark condition. Therefore, the measurement process and the light emission process for obtaining a characteristic value are progressed even when the probe leading end does not appropriately make contact with body tissue. Therefore, an appropriate measurement value may not be obtained. In this regard, according to the third embodiment, the measurement process and the light emission process for obtaining a characteristic value are performed only when the projection length of the probe from the leading end of the insertion portion of the endoscope is set to a level capable of determining that the measurement value can be appropriately obtained. Therefore, it is possible to more reliably obtain only an appropriate measurement value.

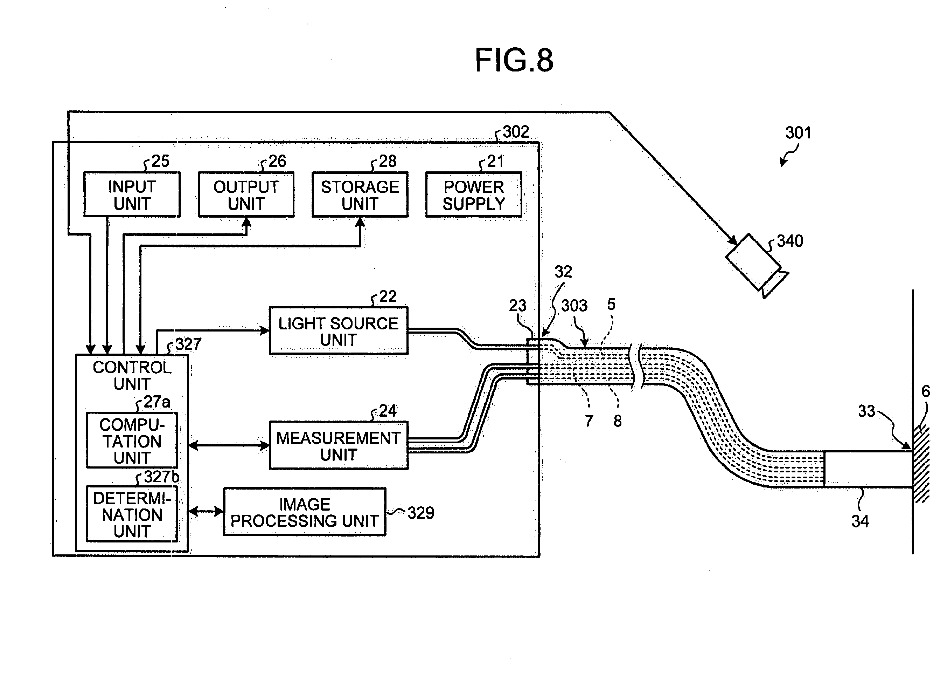

FIG. 8 is a schematic diagram illustrating a schematic configuration of the optical measurement apparatus according to the third embodiment of the present invention. As illustrated in FIG. 8, an optical measurement apparatus 301 according to the third embodiment has a main unit 302 instead of the main unit 2 of FIG. 1. The optical measurement apparatus 301 has a probe 303 having the same function as that of the probe 3 instead of the probe 3. The main unit 302 further includes an image processing unit 329 and an imaging unit 340 in comparison with the main unit 2 of FIG. 1. The main unit 302 has a control unit 327 that has the same function as that of the control unit 27 instead of the control unit 27 and includes a computation unit 27a and a determination unit 327b.

The imaging unit 340 can be inserted into an inner side of a subject and captures an image at the leading end 33 of the probe 303 projected from the leading end of the insertion portion 12 of the endoscope 10. The position of the imaging unit 340 is fixed relative to the aperture 17 of the leading end of the endoscope 10. Since the optical measurement apparatus 301 is connected to the endoscope system, for example, the imaging unit of the leading end of the insertion portion of the endoscope of the endoscope system may serve as the imaging unit 340 of the optical measurement apparatus 301.

The image processing unit 329 serves as a projection length computation unit that computes the projection length 33 of the probe 303 from the leading end of the insertion portion of the endoscope using the photographic image at the leading end of the probe 303 captured by the imaging unit 340.

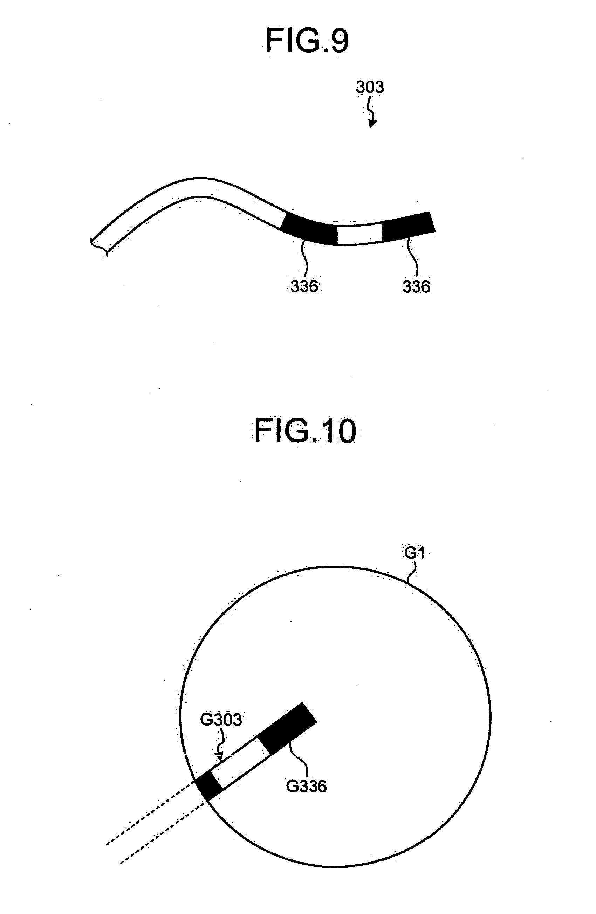

In this case, the leading end of the probe 303 is provided with a plurality of patterns 336 having predetermined regularity as illustrated in FIG. 9. This pattern 336 is a stripe pattern having a ring shape of a predetermined length. The pattern 336 may have a color different from that of the body tissue 6 in order to facilitate contrast with the body tissue 6 which is a red color system. For example, the color may include two colors of black and white.

Since the position of the channel aperture on the image captured by the endoscope 10 is constant for each endoscope 10, the projection initiating position of the probe 303 on the image is already known. In addition, an interval of the pattern 336 is previously stored in the storage unit 28. Therefore, the image processing unit 329 can compute the projection length of the probe 303 by measuring the pattern G336 nearly straightly in a movement direction of the probe 303 on a photographic image G1 (refer to FIG. 10) from the known projection initiating position. The image processing unit 329 divides the area, for example, by binarizing the luminance value of the image data using a predetermined threshold value and determines the image sensing area of the pattern G336. For example, when 200 or more pixels for each R, G, and B (red, green, and blue) are provided in the image sensor of the imaging unit 340, for example, the pixel area is divided into 30 areas, and it is determined whether or not the area is the image sensing area of the pattern G336 based on whether or not the luminance value of each area exceeds a predetermined threshold value.

If the measurement value measured by the measurement unit 24 is equal to or smaller than the predetermined threshold value, and if the projection length of the probe 303 computed by the image processing unit 329 is within a predetermined allowable range at which it can be determined that a constant value can be appropriately obtained, the determination unit 327b causes the light source unit 22 to perform the light emission process for obtaining a characteristic value of the body tissue 6 and causes the measurement unit 24 to perform spectrometry for obtaining a characteristic value of the body tissue 6.

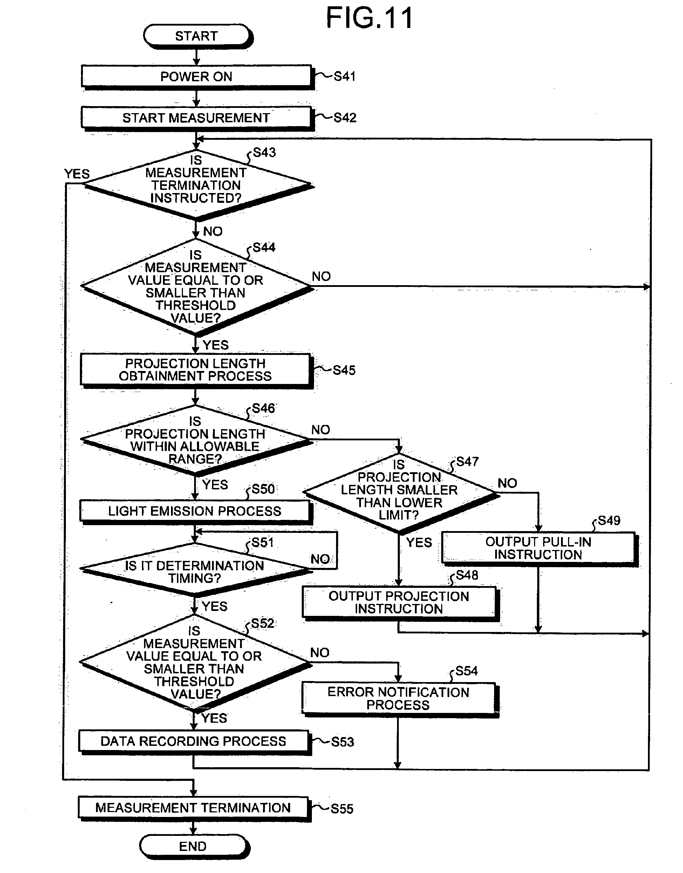

Next, a processing sequence of the optical measurement process of the optical measurement apparatus 301 will be described with reference to FIG. 11. FIG. 11 is a flowchart illustrating an optical measurement processing sequence of the optical measurement apparatus 301 of FIG. 8.

Steps S41 and S42 of FIG. 11 are similar to steps S1 and S2, respectively, of FIG. 5. Subsequently, similar to step S3 of FIG. 5, the determination unit 327b determines whether or not the measurement termination is instructed (step S43). If it is determined that the measurement termination is instructed (Yes in step S43), the measurement process in the measurement unit 24 is terminated (step S55). Otherwise, if it is determined that the measurement termination is not instructed (No in step S43), similar to step S4 of FIG. 5, the determination unit 327b determines whether or not the measurement value output from the measurement unit 24 is equal to or smaller than a predetermined threshold value (step S44). If the determination unit 327b determines that the measurement value output from the measurement unit 24 is not equal to or smaller than the predetermined threshold value (No in step S44), the process returns to step S43.

If the determination unit 327b determines that the measurement value output from the measurement unit 24 is equal to or smaller than the predetermined threshold value (Yes in step S44), the image processing unit 329 obtains an image at the leading end 33 of the probe 303 projected from the leading end of the insertion portion of the endoscope 10 by transmitting the most recently captured photographic image out of the images captured by the endoscope 10 from the connected endoscope system and computes the projection length of the probe 303 from the leading end of the insertion portion of the endoscope 10 (step S45). Subsequently, the determination unit 327b determines whether or not the projection length of the leading end 33 of the probe 303 computed by the image processing unit 329 is within a predetermined allowable range (step S46).

If the determination unit 327b determines that the projection length of the leading end 33 of the probe 303 computed by the image processing unit 329 is not within a predetermined allowable range (No in step S46), the determination unit 327b determines whether or not the projection length is smaller than a lower limit of the allowable range (step S47). If the determination unit 327b determines that the projection length is smaller than the lower limit of the allowable range (Yes in step S47), the projection length of the probe 303 is short. Therefore, the output unit 26 outputs the projection instruction information for instructing projection of the probe 303 from the leading end of the endoscope (step S48), and the process returns to step S43. In addition, if the determination unit 327b determines that the projection length of the probe 303 is not smaller than the lower limit of the allowable range (No in step S47), that is, if the projection length of the probe 303 exceeds the upper limit of the allowable range, the probe 303 is excessively projected. Therefore, the output unit 26 outputs extraction instruction information for instructing to extract the probe 303 into the inner side of the leading end of the endoscope (step S49), and the process returns to step S43. In steps S48 and S49, either an audio output process or a display output process may be performed, or an image may be output and displayed on the display 20 of the connected endoscope system.

Otherwise, if the determination unit 327b determines that the projection length of the leading end 33 of the probe 303 computed by the image processing unit 329 is within a predetermined allowable range (Yes in step S46), it can be determined that an appropriate measurement value can be obtained. Therefore, similar to step S5 of FIG. 5, the light emission process for obtaining a characteristic value of the body tissue 6 is performed in the light source unit 22 (step S50).

Then, similar to step S6 of FIG. 5, the determination unit 327b determines whether or not it is a determination timing for determining whether or not the record of the measurement result measured during the light emission process is appropriate (step S51). If the determination unit 327b determines that it is not the determination timing (No in step S51), the determination process of step S51 is repeated. If the determination unit 327b determines that it is the determination timing (Yes in step S51), similar to step S7 of FIG. 5, it is determined whether or not the measurement value output from the measurement unit 24 during the determination timing is equal to or smaller than a predetermined threshold value (step S52).

If the determination unit 327b determines that the measurement value output from the measurement unit 24 during the determination timing is equal to or smaller than the predetermined threshold value (Yes in step S52), similar to step S8 of FIG. 5, a data recording process is performed for the spectrometric result measured by the measurement unit 24 during the light emission process (step S53). Otherwise, if the determination unit 327b determines that the measurement value output from the measurement unit 24 during the determination timing is not equal to or smaller than the predetermined threshold value (No in step S52), similar to step S9 of FIG. 5, an error notification process for causing the output unit 26 to notify a fact that the obtained measurement value is not valid is performed (step S54). In addition, after step S53 or S54 is terminated, the process returns to step S43 so that the determination unit 327b determines whether or not the measurement termination is instructed.

In this manner, according to the third embodiment, the measurement process and the light emission process for obtaining a characteristic value are performed only when the projection length of the probe from the leading end of the insertion portion of the endoscope is set to a level capable of determining that a measurement value can be appropriately obtained. Therefore, it is possible to more reliably obtain only an appropriate measurement value.

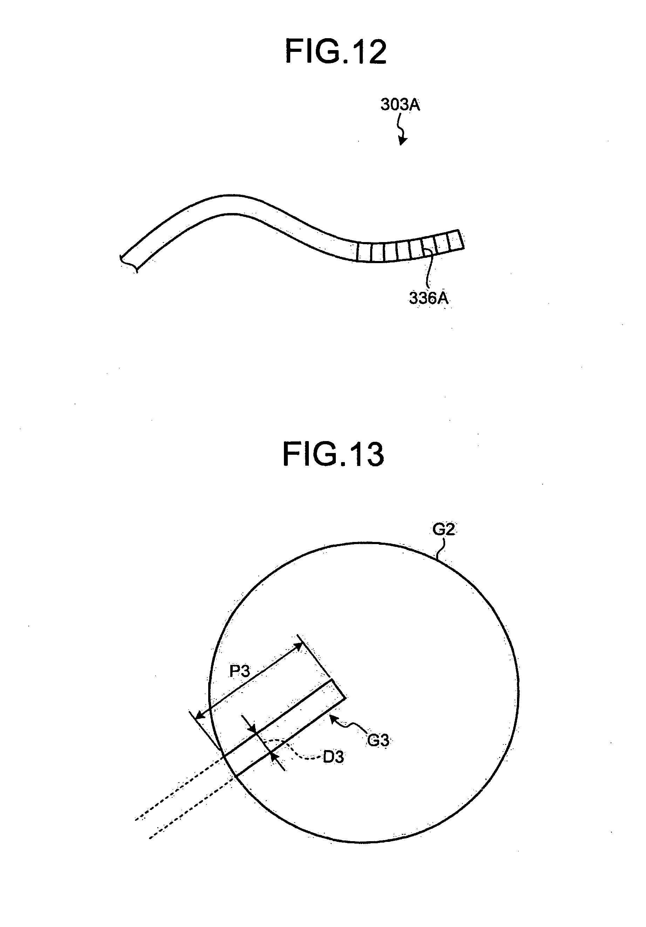

In the third embodiment, the pattern 336 may not be limited to the stripe pattern of FIG. 9. As in a probe 303A of FIG. 12, a memory pattern 336A or a gray-code pattern may be used. As in a concavo-convex pattern in which unevenness is formed in a regular manner, a shape pattern having constant regularity may be formed in the probe leading end.

Since the diameter of the probe leading end is constant in each probe, it is already known. In this regard, the diameter of the probe leading end may be stored in the storage unit 28, and the image processing unit 329 may detect a probe area G3 in a photographic image G2 (refer to FIG. 13) and then compute the projection length of the endoscope 10 of the probe 3 based on the diameter of the probe leading end stored in the storage unit 28 and a ratio between a diameter D3 of the probe G3 viewed on the photographic image G2 and a length P3 of the probe G3 viewed on the photographic image G2. For example, if the actual probe 3 has a diameter of 3 mm, and a ratio between the diameter of the probe on the photographic image and the length of the probe is set to 1:10, the projection length of the probe leading end may be computed as 30 mm. In this case, if the leading end of the probe 3 is provided with a color different from that of the body tissue 6 in order to facilitate contrast with the body tissue 6, it is possible to compute the projection length of the probe 3 without providing the aforementioned patterns 336 and 336A.

### (Fourth Embodiment)

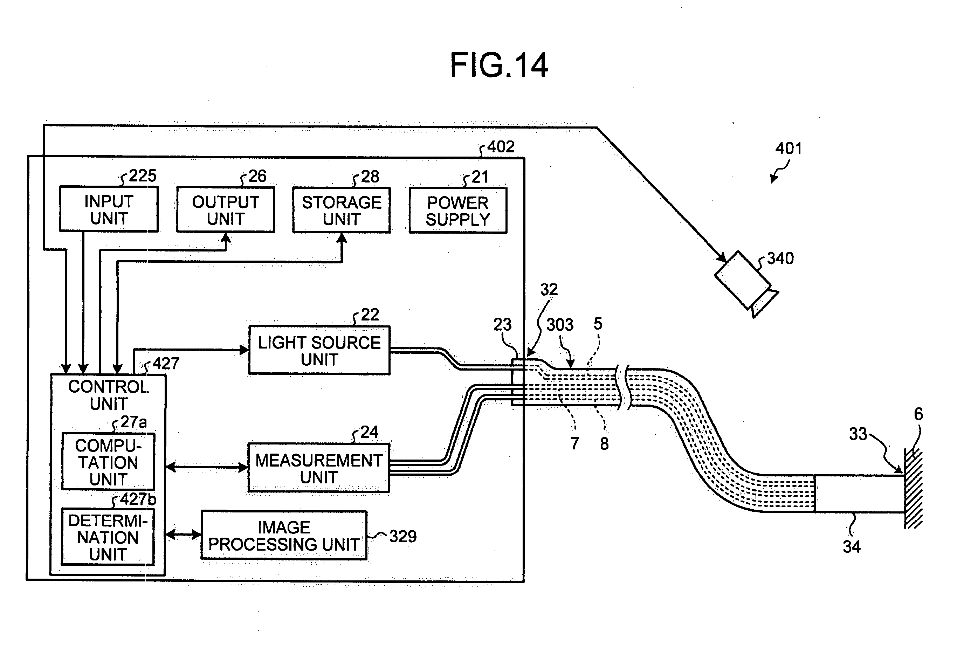

Next, the fourth embodiment will be described. In the fourth embodiment, the third embodiment is applied to the second embodiment. FIG. 14 is a schematic diagram illustrating a schematic configuration of the optical measurement apparatus according to the fourth embodiment of the present invention.

As illustrated in FIG. 14, an optical measurement apparatus 401 according to the fourth embodiment has a main unit 402 instead of the main unit 202 of FIG. 6. The main unit 402 further includes an image processing unit 329 and an imaging unit 340 illustrated in FIG. 8 in comparison with the main unit 202 of FIG. 6. In comparison with the main unit 202 of FIG. 6, the main unit 402 has a control unit 427 that has the same function as that of the control unit 227 and includes a computation unit 27a and a determination unit 427b instead of the control unit 227. If instruction information for instructing to obtain data for obtaining a characteristic value of the body tissue 6 is input by the input unit 225, if it is determined that the measurement value measured by the measurement unit 24 is equal to or smaller than a predetermined threshold value, if the measurement value measured by the measurement unit 24 is equal to smaller than a predetermined threshold value, and if the projection length of the probe 303 computed by the image processing unit 329 is within a predetermined allowable range at which it can be determined that a constant value can be appropriately obtained, the determination unit 427b causes the light source unit 22 to perform a light emission process for obtaining a characteristic value of the body tissue 6 and causes the measurement unit 24 to perform spectrometry for obtaining a characteristic value of the body tissue 6.

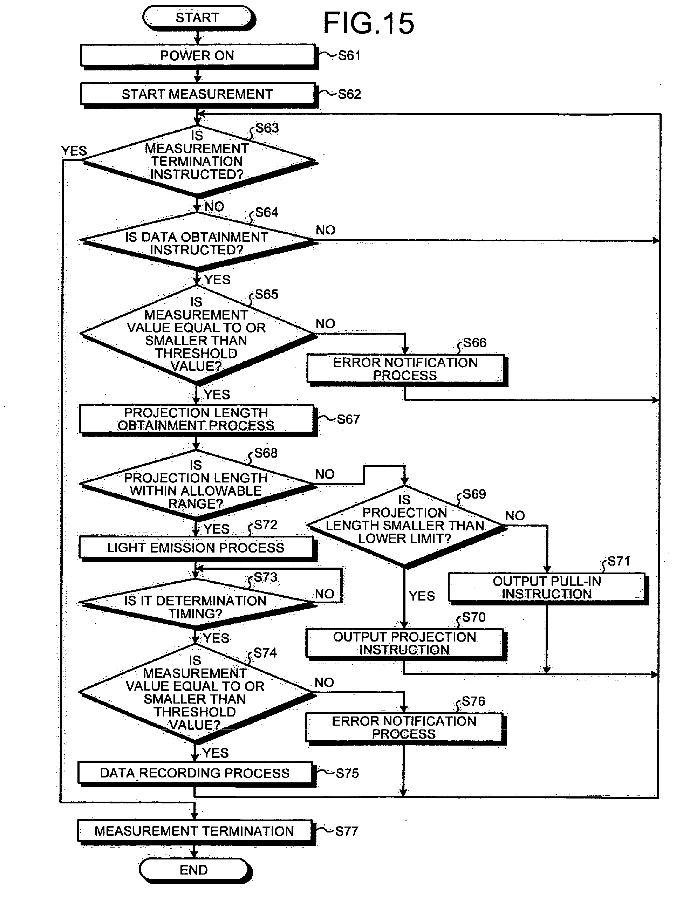

Next, a processing sequence of the optical measurement process of the optical measurement apparatus 401 will be described with reference to FIG. 15. FIG. 15 is a flowchart illustrating an optical measurement processing sequence of the optical measurement apparatus 401 of FIG. 14.

Steps S61 and S62 of FIG. 15 are similar to steps S1 and S2, respectively, of FIG. 5. Subsequently, similar to step S3 of FIG. 5, the determination unit 427b determines whether or not the measurement termination is instructed (step S63). If it is determined that the measurement termination is instructed (Yes in step S63), the measurement process in the measurement unit 24 is terminated (step S77). Otherwise, if it is determined that the measurement termination is not instructed (No in step S63), similar to step S24 of FIG. 7, the determination unit 427b determines whether or not a data obtainment instruction for obtaining a characteristic value is input (step S64). If the determination unit 427b determines that the data obtainment instruction for obtaining a characteristic value is not input (No in step S64), the process returns to step S63.

If the determination unit 427b determines that the data obtainment instruction for obtaining characteristic value is input (Yes in step S64), similar to step S4 of FIG. 5, it is determined whether or not the measurement value output from the measurement unit 24 is equal to or smaller than a predetermined threshold value (step S65). If the determination unit 427b determines that the measurement value output from the measurement unit 24 is not equal to or smaller than the predetermined threshold value (No in step S65), the error notification process similar to that of step S26 of FIG. 7 is performed (step S66), and then, the process returns to step S63.

If the determination unit 427b determines that the measurement value output from the measurement unit 24 is equal to or smaller than the predetermined threshold value (Yes in step S65), similar to step S45 of FIG. 11, the image processing unit 329 computes the projection length from the leading end of the probe 303 in the insertion portion of the endoscope (step S67). Subsequently, the determination unit 427b determines whether or not the projection length of the leading end of the probe 303 computed by the image processing unit 329 is within a predetermined allowable range (step S68).

If the determination unit 427b determines that the projection length of the leading end of the probe 303 computed by the image processing unit 329 is not within the predetermined allowable range (NO in step S68), the determination unit 427b determines whether or not the projection length is smaller than a lower limit of the allowable range (step S69). If the determination unit 427b determines that the projection length is smaller than the lower limit of the allowable range (Yes in step S69), similar to step S48 of FIG. 11, the output unit 26 outputs projection instruction information (step S70), and the process returns to step S63. In addition, if the determination unit 427b determines that the projection length of the probe 303 is not smaller than the lower limit of the allowable range (No in step S69), similar to step S49 of FIG. 11, the output unit 26 outputs extraction instruction information (step S71), and the process returns to step S63.

Otherwise, if the determination unit 427b determines that the projection length of the leading end of the probe 303 computed by the image processing unit 329 is within the predetermined allowable range (Yes in step S68), the light source unit 22 performs a light emission process for obtaining a characteristic value of the body tissue 6 (step S72), and the determination unit 427b determines whether or not it is a determination timing for determining whether or not the record of the measurement result measured during the light emission process is appropriate (step S73). If the determination unit 427b determines that it is not the determination timing (No in step S73), the determination process of step S73 is repeated. If the determination unit 427b determines that it is the determination timing (Yes in step S73), similar to step S7 of FIG. 5, it is determined whether or not the measurement value output from the measurement unit 24 during the determination timing is equal to or smaller than a predetermined threshold value (step S74).

If the determination unit 427b determines that the measurement value output from the measurement unit 24 during the determination timing is equal to or smaller than a predetermined threshold value (Yes in step S74), similar to step S8 of FIG. 5, the data recording process is performed for the spectrometric result measured by the measurement unit 24 during the light emission process (step S75). Otherwise, if the determination unit 427b determines that the measurement value output from the measurement unit 24 during the determination timing is not equal to or smaller than the predetermined threshold value (No in step S74), similar to step S9 of FIG. 5, the error notification process for causing the output unit 26 to notify a fact that the obtained measurement value is not valid is performed (step S76). In addition, after step S75 or S76 is terminated, the process returns to step S63 so that the determination unit 427b determines whether or not the measurement termination is instructed.

In this manner, according to the fourth embodiment, even when data obtainment for obtaining a characteristic value is instructed through manipulation of the input unit 225 from an operator, the measurement process and the light emission process for obtaining a characteristic value are performed only when the projection length of the probe 303 from the leading end of the insertion portion 12 of the endoscope 10 is set to a level capable of determining that the measurement value can be appropriately obtained. Therefore, it is possible to more reliably obtain an appropriate measurement value.

### Reference Signs List

1, 201, 301, 401 optical measurement apparatus
2, 202, 302, 402 main unit
3, 303, 303A probe
5 irradiation fiber
6 body tissue
7, 8 light receiving fiber
10 endoscope
12 insertion portion
13 manipulation unit
14 universal cord
15 probe channel insertion hole
16 leading end portion
17 aperture
18 light source device
19 signal processor
20 display
21 power supply
22 light source unit
23 connector
24 measurement unit
25, 225 input unit
26 output unit
27, 227, 327, 427 control unit
27a computation unit
27b, 227b, 327b, 427b determination unit
28 storage unit
329 image processing unit
340 imaging unit

## Claims

1. An optical measurement apparatus (1, 201, 301, 401) that performs spectrometry of returned light reflected or scattered by body tissue (6) to obtain a characteristic value of the body tissue, the optical measurement apparatus comprising:
a probe (3, 303, 303A) having an irradiation fiber (5) that propagates light supplied from a base end and irradiates the light from a leading end and a plurality of light receiving fibers (7,8) that propagate light incident from leading ends and output the light from base ends;
a light source unit (22) that generates white light to be irradiated onto the body tissue and supplies the white light to the irradiation fiber;
a measurement unit (24) that performs spectrometry for the returned light from the body tissue output from each of the light receiving fibers at a predetermined measurement timing;
a determining unit (27b, 227b, 327b, 427b) that determines whether or not a measurement value that is measured, when the light source unit does not perform light emission, by the measurement unit is equal to or smaller than a predetermined threshold value; and
a control unit (27, 227, 327, 427) that causes the light source unit to perform a light emission process for obtaining a characteristic value of the body tissue for a predetermined time and causes the measurement unit to perform a spectrometry process for obtaining a characteristic value of the body tissue for the predetermined time if the determining unit determines that the measurement value measured by the measurement unit is equal to or smaller than the predetermined threshold value.

2. The optical measurement apparatus according to claim 1, further comprising a storage unit (28) that stores data for obtaining a characteristic value of the body tissue,
wherein the control unit causes the storage unit to store the spectrometric result measured by the measurement unit as data for a characteristic value of the body tissue if it is determined that the measurement value, initially measured by the measurement unit after the light source unit completes the light emission process for obtaining a characteristic value, is equal to or smaller than the predetermined threshold value.

3. The optical measurement apparatus according to claim 1, further comprising an input unit (25, 225) that inputs instruction information for instructing to obtain data for obtaining a characteristic value of the body tissue,
wherein the control unit determines whether or not the measurement value measured by the measurement unit is equal to or smaller than a predetermined threshold value when the instruction information is input by the input unit.

4. The optical measurement apparatus according to claim 1, wherein the probe is inserted into an insertion portion (12) of an endoscope (70) inserted into an inner side of a subject, and a leading end is projected from the endoscope.

5. The optical measurement apparatus according to claim 4, further comprising:
an imaging unit that captures an image at the leading end of the probe projected from the endoscope; and
a projection length computation unit that computes a projection length of the probe from the endoscope using a photographic image of the leading end of the probe captured by the imaging unit,
wherein the control unit causes the light source unit to perform the light emission process and causes the measurement unit to perform the spectrometry, if the measurement value measured by the measurement unit is equal to or smaller than a predetermined threshold value, and the projection length of the probe computed by the projection length computation unit is within a predetermined allowable range.

6. The optical measurement apparatus according to claim 5, wherein a plurality of patterns having predetermined regularity are formed in the leading end of the probe, and
the projection length computation unit computes the projection length of the probe from the endoscope by measuring the pattern viewed on the photographic image.

7. The optical measurement apparatus according to claim 5, wherein the storage unit stores a diameter of the probe, and
the projection length computation unit computes the projection length of the probe from the endoscope based on the diameter of the probe stored in the storage unit and a ratio between a diameter of the probe viewed on the photographic image and a length of the probe viewed on the photographic image.

## Patentansprüche

1. Optisches Messgerät (1, 201, 301, 401), das Spektrometrie von zurückgesandtem Licht, das an Körpergewebe (6) reflektiert oder gestreut wird, ausführt, um einen charakteristischen Wert des Körpergewebes zu erhalten, wobei das optische Messgerät umfasst:
eine Sonde (3, 303, 303A), die eine Strahlungsfaser (5), welche Licht, das von einem Basisende zugeführt wird, weiterleitet und das Licht von einem Führungsende ausstrahlt, und eine Mehrzahl von Licht empfangenden Fasern (7,8) umfasst, die Licht weiterleiten, das von Führungsenden einfällt und das Licht von Basisenden aufgeben;
eine Lichtquelleneinheit (22), die weißes Licht generiert, das dazu vorgesehen ist, auf das Körpergewebe gestrahlt zu werden, und das weiße Licht der Strahlungsfaser zuführt;
eine Messeinheit (24), die Spektrometrie für das zurückgesandte Licht von dem Körpergewebe durchführt, welches von jeder der Licht empfangenden Fasern bei einer vorbestimmten Messzeit ausgegeben wird;
eine Bestimmungseinheit (27b, 227b, 327b, 427b), die bestimmt, ob ein Messwert, der von der Messeinheit gemessen wird, wenn die Lichtquelleneinheit keine Lichtemission ausführt, gleich einem vorbestimmten Schwellwert oder kleiner als ein vorbestimmter Schwellwert ist oder nicht; und
eine Steuereinheit (27, 227, 327, 427), die die Lichtquelleneinheit veranlasst, einen Lichtemissionsprozess auszuführen, um einen charakteristischen Wert des Körpergewebes für eine vorbestimmte Zeit zu erhalten und die die Messeinheit veranlasst, einen Spektrometrieprozess auszuführen, um einen charakteristischen Wert des Körpergewebes für eine vorbestimmte Zeit zu erhalten, wenn die Bestimmungseinheit bestimmt, dass der von der Messeinheit gemessene Messwert gleich dem vorbestimmten Schwellwert oder kleiner als der vorbestimmte Schwellwert ist.

2. Optisches Messgerät gemäß Anspruch 1, das ferner eine Speichereinheit (28) umfasst, die Daten speichert, um einen charakteristischen Wert des Körpergewebes zu erhalten,
wobei die Steuereinheit die Speichereinheit veranlasst, das von der Messeinheit gemessene spektrometrische Ergebnis als Daten für einen charakteristischen Wert des Körpergewebes zu speichern, wenn bestimmt wird, dass der Messwert, der anfänglich von der Messeinheit gemessen wird, nachdem die Lichtquelleneinheit den Lichtemissionsprozess zum Erhalten eines charakteristischen Werts beendet, gleich dem vorbestimmten Schwellwert oder kleiner als der vorbestimmte Schwellwert ist.

3. Optisches Messgerät gemäß Anspruch 1, das ferner eine Eingabeeinheit (25, 225) umfasst, die Anweisungsinformationen eingibt, um anzuweisen, Daten zum Erhalten eines charakteristischen Werts des Körpergewebes zu erhalten,
wobei die Steuereinheit bestimmt, ob der Messwert, der von der Messeinheit gemessen wird, gleich einem vorbestimmten Schwellwert oder kleiner als ein vorbestimmter Schwellwert ist oder nicht, wenn die Anweisungsinformationen von der Eingabeeinheit eingegeben werden.

4. Optisches Messgerät gemäß Anspruch 1, wobei die Sonde in einen Einführabschnitt (12) eines Endoskops (10) eingeführt ist, das in ein Inneres eines Subjekts eingeführt ist und ein Führungsende von dem Endoskop vorsteht.

5. Optisches Messgerät gemäß Anspruch 4, das ferner umfasst:
eine Bildgebungseinheit, die ein Bild an dem Führungsende der Sonde, die von dem Endoskop vorsteht, aufnimmt; und
eine Vorstehlängenberechnungseinheit, die eine Vorstehlänge der Sonde von dem Endoskop unter Nutzung eines photographischen Bilds des Führungsendes der Sonde berechnet, das von der Bildgebungseinheit aufgenommen wird,
wobei die Steuereinheit die Lichtquelleneinheit veranlasst, den Lichtemissionsprozess auszuführen, und die Messeinheit veranlasst, die Spektrometrie auszuführen, wenn der von der Messeinheit gemessene Messwert gleich dem vorbestimmten Schwellwert oder kleiner als der vorbestimmte Schwellwert ist und die Vorstehlänge der Sonde, die von der Vorstehlängenberechnungseinheit berechnet wird, innerhalb eines vorbestimmten zulässigen Bereichs liegt.

6. Optisches Messgerät gemäß Anspruch 5, wobei eine Mehrzahl von Mustern, die eine vorbestimmte Regularität aufweisen, in dem Führungsende der Sonde ausgebildet sind, und
die Vorstehlängenberechnungseinheit die Vorstehlänge der Sonde von dem Endoskop berechnet, indem sie das Muster, das auf dem photographischen Bild betrachtet wird, misst.

7. Optisches Messgerät gemäß Anspruch 5, wobei die Speichereinheit einen Durchmesser der Sonde speichert, und
die Vorstehlängenberechnungseinheit die Vorstehlänge der Sonde von dem Endoskop auf der Basis des Durchmessers der Sonde, der in der Speichereinheit gespeichert ist, und einem Verhältnis zwischen einem Durchmesser der Sonde, der auf dem photographischen Bild betrachtet wird, und einer Länge der Sonde, die auf dem photographischen Bild betrachtet wird, berechnet.

## Revendications

1. Appareil (1, 201, 301, 401) de mesure optique qui effectue une spectrométrie d'une lumière renvoyée réfléchie ou diffusée par un tissu (6) corporel pour obtenir une valeur caractéristique du tissu corporel, l'appareil de mesure optique comprenant :
une sonde (3, 303, 303A) ayant une fibre (5) de projection énergétique qui propage une lumière délivrée d'une extrémité de base et projette la lumière à partir d'une extrémité avant et une pluralité de fibres (7, 8) de réception de lumière qui propagent une lumière incidente à partir d'extrémités avant et délivrent la lumière en sortie à partir d'extrémités de base ;
une unité (22) de source de lumière qui génère une lumière blanche destinée à être projetée sur le tissu corporel et délivre la lumière blanche à la fibre de projection énergétique ;
une unité (24) de mesure qui effectue une spectrométrie pour la lumière renvoyée du tissu corporel délivrée en sortie de chacune des fibres de réception de lumière à un moment de mesure prédéterminé ;
une unité (27b, 227b, 327b, 427b) de détermination qui détermine si une valeur de mesure qui est mesurée, lorsque l'unité de source de lumière n'effectue pas une émission de lumière, par l'unité de mesure est égale ou inférieure à une valeur de seuil prédéterminée ; et
une unité (27, 227, 327, 427) de commande qui fait que l'unité de source de lumière met en oeuvre un processus d'émission de lumière pour obtenir une valeur caractéristique du tissu corporel sur une durée prédéterminée et fait que l'unité de mesure met en oeuvre un processus de spectrométrie pour obtenir une valeur caractéristique du tissu corporel sur la durée prédéterminée si l'unité de détermination détermine que la valeur de mesure mesurée par l'unité de mesure est égale ou inférieure à la valeur de seuil prédéterminée.

2. Appareil de mesure optique selon la revendication 1, comprenant en outre une unité (28) de stockage qui stocke des données pour obtenir une valeur caractéristique du tissu corporel,
dans lequel l'unité de commande fait que l'unité de stockage stocke le résultat de spectrométrie mesuré par l'unité de mesure comme données pour une valeur caractéristique du tissu corporel s'il est déterminé que la valeur de mesure, initialement mesurée par l'unité de mesure après que l'unité de source de lumière a terminé le processus d'émission de lumière pour obtenir une valeur caractéristique, est égale ou inférieure à la valeur de seuil prédéterminée.

3. Appareil de mesure optique selon la revendication 1, comprenant en outre une unité (25, 225) d'entrée qui entre des informations d'instruction pour indiquer d'obtenir des données pour obtenir une valeur caractéristique du tissu corporel,
dans lequel l'unité de commande détermine si la valeur de mesure mesurée par l'unité de mesure est égale ou inférieure à une valeur de seuil prédéterminée lorsque les informations d'instruction sont entrées par l'unité d'entrée.

4. Appareil de mesure optique selon la revendication 1, dans lequel la sonde est insérée dans une partie (12) d'insertion d'un endoscope (10) inséré dans un côté intérieur d'un sujet, et une extrémité avant est projetée de l'endoscope.

5. Appareil de mesure optique selon la revendication 4, comprenant en outre :
une unité d'imagerie qui capture une image à l'extrémité avant de la sonde projetée de l'endoscope ; et
une unité de calcul de longueur de projection qui calcule une longueur de projection de la sonde de l'endoscope en utilisant une image photographique de l'extrémité avant de la sonde capturée par l'unité d'imagerie,
dans lequel l'unité de commande fait que l'unité de source de lumière met en oeuvre le processus d'émission de lumière et fait que l'unité de mesure effectue la spectrométrie, si la valeur de mesure mesurée par l'unité de mesure est égale ou inférieure à une valeur de seuil prédéterminée, et si la longueur de projection de la sonde calculée par l'unité de calcul de longueur de projection est à l'intérieur d'une plage autorisée prédéterminée.

6. Appareil de mesure optique selon la revendication 5, dans lequel une pluralité de motifs ayant une régularité prédéterminée sont formés dans l'extrémité avant de la sonde, et
l'unité de calcul de longueur de projection calcule la longueur de projection de la sonde de l'endoscope en mesurant le motif vu sur l'image photographique.

7. Appareil de mesure optique selon la revendication 5, dans lequel l'unité de stockage stocke un diamètre de la sonde, et
l'unité de calcul de longueur de projection calcule la longueur de projection de la sonde de l'endoscope sur la base du diamètre de la sonde stocké dans l'unité de stockage et d'un rapport entre un diamètre de la sonde vu sur l'image photographique et une longueur de la sonde vue sur l'image photographique.
